# FASCICULE DE BREVET EUROPEEN

(11) **EP 3 049 181 B1**
(45) Date de publication et mention de la délivrance du brevet: **18.11.2020**
(21) Numéro de dépôt: 14796200.5
(22) Date de dépôt: 25.09.2014
(51) Int. Cl.: B01J 8/02, B01J 8/18, C07C 1/04, C07C 9/04

(54) **RÉACTEUR DE MÉTHANATION POUR FAIRE RÉAGIR DE L'HYDROGÈNE AVEC AU MOINS UN COMPOSÉ À BASE DE CARBONE ET PRODUIRE DU MÉTHANE ET DE L'EAU**
METHANISIERUNGSREAKTOR ZUR REAKTION VON WASSERSTOFF MIT MINDESTENS EINER KOHLENSTOFFHALTIGEN VERBINDUNG UND HERSTELLUNG VON METHAN UND WASSER
METHANATION REACTOR FOR REACTING HYDROGEN WITH AT LEAST ONE CARBON-BASED COMPOUND AND PRODUCING METHANE AND WATER

(30) Priorité: 26.09.2013 FR 1359313
(43) Date de publication de la demande: 03.08.2016
(73) Titulaire: ENGIE, 92400 Courbevoie (FR)
(72) Inventeur: KARA, Ylmaz, F-95600 Eaubonne (FR); MARCHAND, Bernard, F-75018 Paris (FR)
(74) Mandataire: Cornuejols, Georges
(86) Numéro de dépôt international: PCT/FR2014/052411
(87) Numéro de publication internationale: WO 2015/044601

(56) Documents cités:
- WO-A1-2012/035881
- DE-A1- 2 506 199
- US-A- 4 312 741

## Description

### DOMAINE TECHNIQUE DE L'INVENTION

La présente invention vise un réacteur de méthanation pour faire réagir de l'hydrogène avec un composé à base de carbone et produire du méthane. Elle s'applique notamment à la méthanation industrielle et à la co-génération d'énergie thermique et de méthane.

### ETAT DE LA TECHNIQUE

La méthanation est un procédé industriel de conversion catalytique de l'hydrogène et du monoxyde ou du dioxyde de carbone en méthane.

Selon la nature du composé à base de carbone, la formule de la réaction de méthanation change. Cette formule est, selon le cas :

CO + 3 H₂ → CH₄ + H₂O

CO₂ + 4 H₂ → CH₄ + 2 H₂O

Afin d'optimiser les rendements de cette réaction, un lit de catalyseur est placé dans un réacteur dans lequel a lieu la réaction. Ce lit peut être fixe ou fluidisé. La réaction de méthanation étant fortement exothermique, elle entraîne des besoins importants d'évacuation de la chaleur et donc de refroidissement du réacteur. Un lit de catalyseur fluidisé permet d'homogénéiser la température de la zone réactive. Enfin, la cinétique de cette réaction aux températures habituellement mises en œuvre est élevée, nécessitant, de fait, une masse faible de catalyseur.

Dans des systèmes actuels à lits fixes, appelés « Throughwall Cooled Reactor » (traduit en français par « Réacteur refroidi à travers les parois »), un échange thermique est réalisé par des parois du réacteur refroidies par un fluide de refroidissement. Cependant les surfaces nécessitées pour réaliser l'échange thermique sont importantes et les coûts de fabrication du réacteur sont élevés.

Dans des systèmes actuels à lits fluidisés, un ou plusieurs échangeurs de chaleur sont immergés dans le lit fluidisé à l'intérieur du réacteur. On met alors en circulation dans ces échangeurs de l'eau, de la vapeur d'eau ou une huile thermique, par exemple. Les coefficients d'échange thermique entre la paroi de l'échangeur et le lit fluidisé sont très importants, de l'ordre des coefficients d'échange thermique entre un liquide et une paroi. Cependant, l'utilisation d'huiles thermiques n'est possible que jusqu'à des températures de réaction de l'ordre de 380°C à 400°C. De plus, dans ces systèmes, la taille du réacteur dépend de la taille occupée par chaque échangeur à immerger dans le lit fluidisé. Ces systèmes entraînent un coût de fabrication et une utilisation d'espace non optimisés du réacteur. Par ailleurs, l'efficacité des échanges de chaleur entre le lit et le fluide de refroidissement dépend fortement des conditions de fluidisation.

Dans des systèmes actuels à lits fixes ou à lits fluidisés, l'injection de vapeur en mélange avec l'hydrogène et le composé à base de carbone permet de limiter la forme d'un dépôt de carbone sous forme de coke sur le catalyseur dont l'une des conséquences est une désactivation prématurée du catalyseur. Enfin, les catalyseurs de méthanation étant préférentiellement constitués au moins en partie de nickel, la réaction de méthanation risque d'entrainer la formation de carbonyle, un composé hautement toxique, au contact de parois portées à une température inférieure à 260°C, ce qui complique le système de refroidissement.

On connaît les documents WO2012/035881, US4312741 et DE2506199. Les enseignements de ces documents ne permettent pas de réaliser un refroidissement d'un réacteur de méthanation en limitant la formation de coke ou de carbonyle dans le réacteur.

En particulier, le document WO2012/035881 décrit un réacteur avec des entrées et des sorties susceptible de mettre en œuvre une réaction de méthanation. Cependant, ce réacteur ne comporte pas d'entrée pour injection d'eau dans le réacteur afin de refroidir la réaction chimique.

Le document US4312741 décrit un réacteur de méthanation. Cependant, ce réacteur ne comporte pas d'entrée pour eau en phase liquide dans le réacteur.

Le document DE2506199 décrit un réacteur de méthanation avec une entrée d'eau au dessus d'un lit catalytique contenu dans le réacteur. Ce système présente l'inconvénient de ne pas limiter la formation de coke ou de carbonyle dans le réacteur lors de l'injection d'eau.

### OBJET DE L'INVENTION

La présente invention vise à remédier à tout ou partie de ces inconvénients.

A cet effet, la présente invention vise, selon un premier aspect, un réacteur de méthanation pour faire réagir du dihydrogène avec au moins un composé à base de carbone et produire du méthane selon la revendication 10.

Bien que l'introduction, dans le lit fluidisé du réacteur, d'un produit de la réaction, en plus des réactifs soit, à priori, l'inverse de ce que l'homme du métier réalise pour obtenir un bon rendement de la réaction, les inventeurs ont déterminé que cette introduction est favorable en termes de contrôle de la température dans le réacteur, de dimensions du réacteur, de complexité du réacteur de coût de fabrication et de maintenance du réacteur dans la mesure où ce réactif est introduit en phase liquide. Cette introduction permet aussi de réduire, voire d'éliminer la production de carbonyle. Enfin, cette introduction permet de limiter la formation de coke en surface de catalyseur ; l'eau injectée est vaporisée au contact du lit chaud.

Grâce aux caractéristiques du réacteur objet de la présente invention, la taille du réacteur peut être définie en fonction de la quantité de lit catalytique à contenir pour assurer la conversion de l'hydrogène et du composé à base de carbone. De plus, l'eau introduite est utilisée par la réaction de méthanation par le biais de la réaction dite de « Water Gas Shift » (traduit en français par « Réaction du gaz à l'eau ») dans laquelle du monoxyde de carbone et de l'eau donnent du dioxyde de carbone et du dihydrogène. Enfin, ces dispositions permettent d'obtenir, à la sortie du réacteur, une composition molaire en eau du mélange de vapeur d'eau et de méthane supérieure à 50%.

Dans des modes de réalisation, l'entrée de chaque composé à base de carbone et du dihydrogène est réalisée dans le lit.

Ces modes de réalisation permettent d'augmenter les rendements de la réaction entre chaque composé à base de carbone et le dihydrogène dans le catalyseur. L'entrée d'eau dans le lit permet de le refroidir sans risquer que des carbonyles ne se forment au contact de parois.

Dans des modes de réalisation, l'entrée d'eau est plus proche de la base du corps creux que l'entrée de chaque composé à base de carbone et du dihydrogène.

Ces modes de réalisation permettent d'éviter le dépôt de coke sur le catalyseur.

Dans des modes de réalisation, chaque composé à base de carbone est un gaz, le réacteur comportant au moins une buse d'injection d'eau et au moins une buse d'injection d'un gaz comportant le gaz à base de carbone et du dihydrogène, au moins une buse d'injection d'eau étant positionnée en dessous d'au moins une buse d'injection du gaz.

Ces modes de réalisation, permettent une injection optimisée de gaz et d'eau dans le corps creux du réacteur.

Le réacteur objet de la présente invention comporte un moyen de condensation de vapeur d'eau présente en aval de la sortie de méthane et d'eau.

Ces modes de réalisation permettent de séparer, par condensation, l'eau du méthane en aval de la sortie de méthane. De plus, ces modes de réalisation permettent la récupération d'eau condensée.

Le réacteur objet de la présente invention comporte un circuit de transport de l'eau condensée jusqu'à l'entrée d'injection d'eau de refroidissement.

Ces modes de réalisation permettent de réaliser un recyclage de l'eau créée par la réaction de méthanation pour refroidir cette réaction.

Dans des modes de réalisation, le réacteur objet de la présente invention comporte, en aval de la sortie de méthane et d'eau, un moyen de séparation gaz - solide.

Ces modes de réalisation permettent d'assurer que le méthane et l'eau sortis du dispositif sont en phase gazeuse et d'éviter la présence de solides en sortie du dispositif, comme par exemple des particules du lit catalytique.

Le réacteur objet de la présente invention comporte un capteur de température dans le réacteur et un moyen de régulation du débit d'eau introduit dans le corps creux en fonction de la température mesurée par le capteur de température.

Ces modes de réalisation permettent d'optimiser la température de réaction de manière à obtenir un rendement optimal de méthane en fonction du composé à base de carbone introduit dans le réacteur.

Dans des modes de réalisation, le réacteur objet de la présente invention comporte en aval de la sortie de méthane et d'eau un échangeur de chaleur configuré pour refroidir le méthane et l'eau et pour co-générer de l'énergie thermique au cours de l'échange de chaleur réalisé.

Ces modes de réalisation permettent de co-générer de l'énergie thermique et du méthane, à partir du mélange de vapeur d'eau et de méthane en sortie du corps creux.

Dans des modes de réalisation, la quantité d'eau introduite dans le corps creux par l'entrée d'injection d'eau est supérieure à 75 % de la quantité d'eau sortant du corps creux. Le refroidissement dû à l'eau introduite est ainsi particulièrement important.

Selon un deuxième aspect, la présente invention vise un procédé de méthanation pour faire réagir du dihydrogène avec au moins un composé à base de carbone et produire du méthane selon la revendication 1.

Les avantages, buts et caractéristiques particulières de ce procédé étant similaires à ceux du réacteur de méthanation objet de la présente invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du réacteur de méthanation et du procédé de méthanation objets de la présente invention, en regard des dessins annexés, dans lesquels :
- la figure 1 représente, schématiquement, un mode de réalisation particulier du réacteur de méthanation objet de la présente invention et
- la figure 2 représente, sous forme d'un logigramme, des étapes d'un mode de réalisation particulier du procédé de méthanation objet de la présente invention.

### DESCRIPTION D'EXEMPLES DE REALISATION DE L'INVENTION

La présente description est donnée à titre non limitatif.

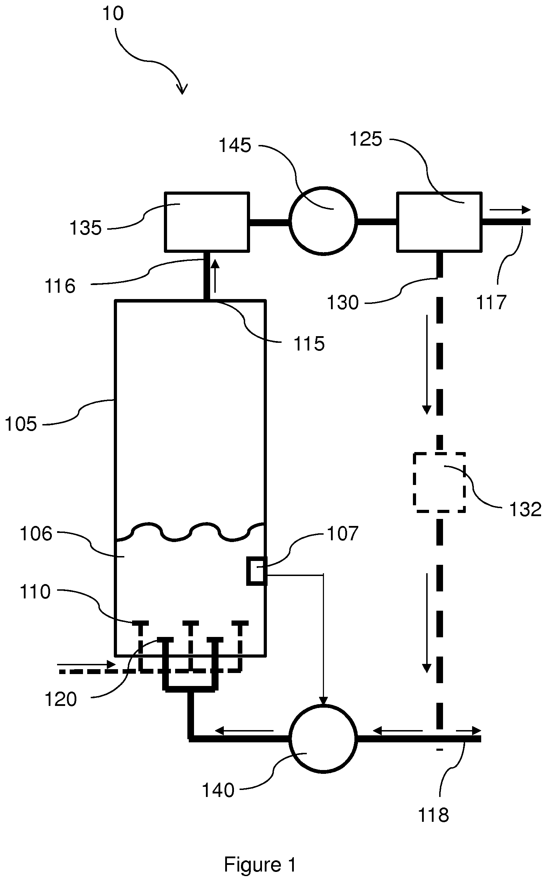

On note, dès à présent, que la figure 1 n'est pas à l'échelle.

On observe, sur la figure 1, un mode de réalisation particulier du réacteur 10 objet de la présente invention. Ce réacteur 10 comporte :
- un corps creux 105 configuré pour recevoir un lit fluidisé de particules catalytiques 106 et qui comporte au moins une buse 110 d'injection d'un composé à base de carbone et de dihydrogène, et au moins une buse 120 d'injection d'eau de refroidissement,
- une sortie de méthane et d'eau 115,
- un moyen de séparation gaz - solide 135 du méthane issu de la réaction de méthanation,
- un échangeur de chaleur 145 configuré pour refroidir le méthane et l'eau et pour co-générer de l'énergie thermique au cours de l'échange de chaleur réalisé,
- un moyen de condensation 125 de vapeur d'eau présente en aval de la sortie de méthane 115,
- un circuit 130 de transport de l'eau condensée jusqu'à une buse d'injection d'eau de refroidissement 120 et
- un moyen de régulation 140 du débit d'eau introduit dans le corps creux 105 en fonction de la température mesurée dans le réacteur 10 par un capteur de température 107.

Le corps creux 105 est, par exemple, un cylindre de révolution métallique fermé à ses extrémités. Ce corps creux 105 est partiellement rempli d'un lit de catalyseur fluidisé. Par effet de la pesanteur, ce catalyseur se situe près de la base du corps creux 105. Ce corps creux 105 comporte au moins une buse 110 d'injection du composé à base de carbone et de dihydrogène permettant l'introduction dans le lit fluidisé du composé à base de carbone et du dihydrogène. Préférentiellement, le composé à base de carbone est du monoxyde ou du dioxyde de carbone sous forme gazeuse.

De plus, ce corps creux 105 comporte au moins une buse 120 d'injection d'eau de refroidissement. La sortie de chaque buse 120 d'injection d'eau de refroidissement est préférentiellement plus proche de la base du corps creux 105 que la sortie de chaque buse 110 d'injection du composé à base de carbone. De cette manière, l'eau injectée est très rapidement portée à l'état de vapeur lors du contact avec le lit fluidisé en absorbant une chaleur latente de changement d'état. La majeure partie de l'échange de chaleur entre l'eau injectée et le lit fluidisé étant réalisée à proximité de la buse d'injection d'eau de refroidissement 120, la température du lit fluidisé au niveau de la buse 110 d'injection du composé à base de carbone est supérieure à 260°C, ce qui réduit, voie élimine, la formation de carbonyle.

Préférentiellement, la quantité d'eau introduite par les buses 120 d'injection d'eau est supérieure à 75 % de la quantité d'eau sortant du corps creux, plus préférentiellement supérieure à 80 % et, encore plus préférentiellement, supérieure à 85 %. L'injection d'eau, par les buses 120 d'injection, est préférentiellement réalisée directement dans le lit fluidisé contenu dans le corps creux 105.

Ce corps creux 105 comporte, enfin, une sortie de méthane et de vapeur d'eau 115 qui débouche sur une canalisation 116. Cette canalisation mène le méthane et la vapeur d'eau à un moyen de séparation gaz - solide 135 du méthane sorti. Ce moyen de séparation gaz - solide 135 est, par exemple, un filtre configuré pour retenir des particules fines de catalyseur pouvant être transportés par le méthane et/ou la vapeur d'eau.

Ce réacteur 10 comporte, de plus, en aval du moyen de séparation gaz - solide 135, un échangeur de chaleur 145 configuré pour refroidir le méthane et l'eau et pour co-générer de l'énergie thermique au cours de l'échange de chaleur réalisé. Cet échangeur 145 est, par exemple, un échangeur à tubes en U. Dans des variantes, cet échangeur 145 est un échangeur parmi :
- échangeur à faisceau tubulaire horizontal,
- échangeur à faisceau tubulaire vertical,
- échangeur à spirales,
- échangeur à plaques,
- échangeur à blocs et
- échangeur à ailettes.

Ce réacteur 10 comporte, de plus, en aval de l'échangeur 145 de chaleur, un moyen de condensation 125 de vapeur d'eau. Ce moyen de condensation 125 est, par exemple, un condenseur à fluides séparés. Dans des variantes, ce moyen de condensation 125 est un condenseur à contact direct entre un fluide réfrigérant et la vapeur à condenser. Dans d'autres variantes, ce moyen de condensation 125 est un échangeur de chaleur à calandre ou à faisceaux tubulaires. Dans ces variantes, l'échangeur 145 et le moyen de condensation 125 sont combinés en un seul dispositif. Le méthane, non condensé, sort par une canalisation 117.

Dans des variantes, en aval du moyen de condensation 125, le réacteur 10 comporte le circuit 130 de transport de l'eau condensée, dont une partie est évacuée par une canalisation de sortie 118 et une partie est transportée jusqu'aux buses 120 d'injection d'eau de refroidissement par la mise en œuvre d'une pompe 132. La proportion d'eau ainsi recyclée est du même ordre de grandeur que le débit de condensat, c'est à dire de l'ordre de 85% à 95% selon la température du moyen de condensation.

Le réacteur 10 comporte, de plus, un moyen de régulation 140 du débit d'eau introduit dans le corps creux 105 en fonction de la température mesurée dans le lit présent dans réacteur 10, par le capteur de température 107. Le moyen de régulation 140 est, par exemple, une vanne contrôlée pneumatiquement ou électroniquement par un circuit électronique (non représenté). Ce circuit électronique reçoit une information représentative de la température à l'intérieur du corps creux 105 et actionne la valve en fonction de l'information reçue pour que le débit d'eau introduit dans le corps creux soit une fonction croissante de la température mesurée. On réalise ainsi une boucle d'asservissement de la température à l'intérieur dans le lit fluidisé de catalyseur du corps creux 105.

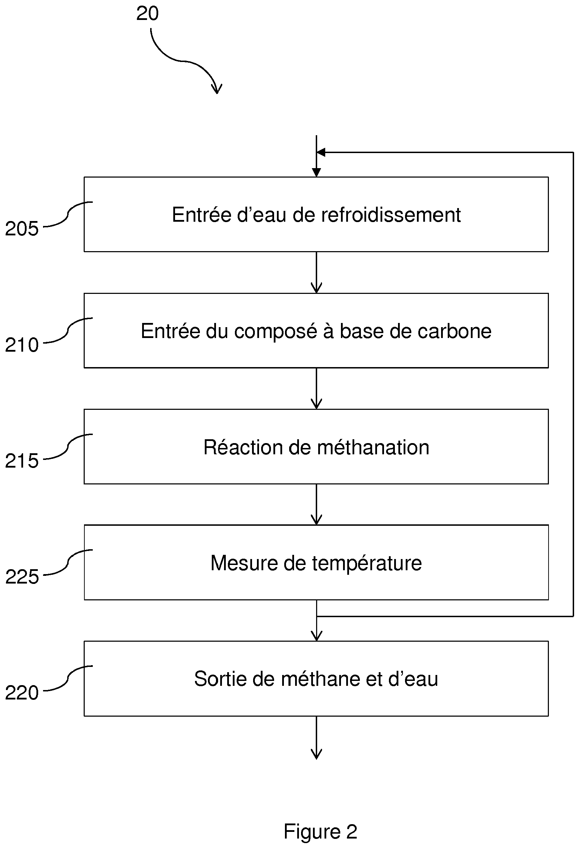

On observe, sur la figure 2, un logigramme d'étapes d'un mode de réalisation particulier du procédé 20 de méthanation objet de la présente invention. Ce procédé 20 comporte :
- une étape 205 d'injection d'eau, en phase liquide, de refroidissement dans un lit fluidisé contenu dans un corps creux lors d'une étape de réaction 215 de méthanation,
- une étape 210 d'entrée du composé à base de carbone et d'hydrogène dans le corps creux configuré pour recevoir un lit fluidisé de particules catalytiques,
- une étape 215 de réaction de méthanation entre de l'hydrogène et le composé à base de carbone pour produire du méthane et de l'eau,
- une étape 225 de mesure de température à l'intérieur du corps creux et
- une étape 220 de sortie de méthane et d'eau.

L'étape 205 d'injection d'eau de refroidissement dans le corps creux est réalisée, par exemple, par la mise en œuvre de buses d'injection d'eau de refroidissement, qui injectent de l'eau au niveau d'un lit de catalyseur fluidisé contenu dans le corps creux.

L'étape 210 d'entrée du composé à base de carbone et d'hydrogène dans le corps creux est réalisée, par exemple, par la mise en œuvre de buses d'injection de monoxyde ou de dioxyde de carbone et de dihydrogène. Ces buses d'injection injectent le gaz au dessus d'au moins une, et préférentiellement de toutes les buses d'injection d'eau de refroidissement.

L'étape 220 de sortie de méthane et d'eau est réalisée, par exemple, par la mise en œuvre d'une canalisation dont une entrée se situe sur une partie supérieure du corps creux.

La mesure de température à l'intérieur du corps creux réalisée au cours de l'étape 225 sert à l'asservissement du débit d'eau introduit dans le corps creux au cours de l'étape 205, ce débit étant une fonction croissante de la température à l'intérieur du corps creux.

Les différentes étapes représentées en figure 2 sont réalisées en permanence et simultanément pendant le fonctionnement nominal du réacteur. Préférentiellement, l'eau introduite dans le corps creux au cours l'étape 205 est de l'eau issue de la réaction refroidie par un condenseur et, éventuellement, un échangeur de chaleur ou un dispositif combinant la fonctionnalité d'un condenseur et d'un échangeur de chaleur.

Comme on le comprend à la lecture de la description qui précède, la présente invention permet de réduire la taille d'un réacteur de méthanation. En effet, l'injection d'eau directement dans le milieu réactionnel permet de ne pas avoir recours à un échangeur de chaleur dont les surfaces d'échanges à utiliser sont importantes. De plus, l'eau injectée est utilisée au sein du réacteur par le biais de la formule de réaction du gaz à l'eau afin d'assurer la présence de dihydrogène dans la réaction de méthanation. De plus, la présence d'un moyen de condensation de l'eau en aval de la sortie de méthane et d'eau permet de recycler l'eau naturellement produite par la réaction de méthanation pour refroidir la réaction à un instant ultérieur. Enfin, la température à l'intérieur du réacteur est asservie par introduction d'eau selon une fonction croissante de la température mesurée dans le réacteur et la production de carbonyle peut être minimisée.

## Revendications

1. Procédé (20) de méthanation pour faire réagir du dihydrogène sous forme gazeuse avec au moins un composé à base de carbone sous forme gazeuse et produire du méthane, qui comporte :
- une étape (210) d'entrée de chaque composé à base de carbone et du dihydrogène dans un corps creux (105) configuré pour recevoir un lit fluidisé de particules catalytiques (106),
- une étape (215) de réaction de méthanation entre l'hydrogène et chaque composé à base de carbone et
- une étape (220) de sortie de méthane et d'eau ;
**caractérisé en ce qu'**il comporte, de plus :
- une étape (205) d'injection d'eau de refroidissement en phase liquide dans le lit fluidisé lors de l'étape de réaction de méthanation,
- une étape de capture de température dans le réacteur et
- une étape de régulation du débit d'eau introduit dans le corps creux en fonction de la température mesurée au cours de l'étape de capture de température.

2. Procédé (20) de méthanation selon la revendication 1, dans lequel au cours de l'étape (210) d'entrée de chaque composé à base de carbone et du dihydrogène, cette entrée est réalisée dans le lit fluidisé de particules catalytiques (106).

3. Procédé (20) de méthanation selon la revendication 2, dans lequel au cours de l'étape (205) d'injection d'eau, l'eau est injectée plus proche de la base du corps creux (105) que l'entrée de chaque composé à base de carbone et du dihydrogène (110).

4. Procédé (20) de méthanation selon l'une des revendications 2 ou 3, dans lequel chaque composé à base de carbone est un gaz, l'étape (210) d'entrée de chaque composé à base de carbone et du dihydrogène dans un corps creux (105) est réalisée par l'intermédiaire d'au moins une buse d'injection de gaz et l'étape (205) d'injection d'eau est réalisée par l'intermédiaire d'une buse d'injection d'eau positionnée en dessous d'au moins une buse d'injection du gaz.

5. Procédé (20) de méthanation selon l'une des revendications 1 à 4, qui comporte une étape de condensation de vapeur d'eau sortie du corps creux (105) au cours de l'étape (220) de sortie de méthane et d'eau.

6. Procédé (20) de méthanation selon la revendication 5, qui comporte une étape d'injection de l'eau condensée dans le lit fluidisé au cours de l'étape (205) d'injection d'eau condensée.

7. Procédé (20) de méthanation selon l'une des revendications 1 à 6, qui comporte une étape de séparation gaz - solide après l'étape (220) de sortie de méthane et d'eau.

8. Procédé (20) de méthanation selon l'une des revendications 1 à 7, qui comporte une étape d'échange de chaleur refroidissant le méthane et l'eau et co-générant de l'énergie thermique après l'étape (220) de sortie de méthane et d'eau.

9. Procédé (20) de méthanation selon l'une des revendications 1 à 8, dans lequel la quantité d'eau introduite dans le corps creux (105) au cours de l'étape (205) d'injection d'eau de refroidissement en phase liquide est supérieure à 75 % de la quantité d'eau sortant du corps creux au cours de l'étape (220) de sortie de méthane et d'eau.

10. Réacteur (10) de méthanation mettant en œuvre le procédé (20) de méthanation selon la revendication 6, en faisant réagir du dihydrogène sous forme gazeuse avec au moins un composé à base de carbone sous forme gazeuse et produire du méthane, qui comporte :
- un corps creux (105) configuré pour recevoir un lit fluidisé de particules catalytiques (106) et comportant une entrée (110) de chaque composé à base de carbone et du dihydrogène et
- une sortie (115) de méthane et d'eau,
**caractérisé en ce qu'**il comporte, de plus :
- une entrée (120) d'injection d'eau de refroidissement en phase liquide dans le lit fluidisé,
- un capteur (107) de température dans le réacteur,
- un moyen (140) de régulation du débit d'eau introduit dans le corps creux en fonction de la température mesurée par le capteur de température,
- un moyen (125) de condensation de vapeur d'eau présente en aval de la sortie (115) de méthane et d'eau et
- un circuit (130) de transport de l'eau condensée jusqu'à l'entrée (120) d'injection d'eau de refroidissement.

11. Réacteur (10) de méthanation selon la revendication 10, dans lequel l'entrée (110) de chaque composé à base de carbone et du dihydrogène est réalisée dans le lit (106).

12. Réacteur (10) de méthanation selon la revendication 11, dans lequel l'entrée d'eau (120) est plus proche de la base du corps creux (105) que l'entrée de chaque composé à base de carbone et du dihydrogène (110).

13. Réacteur (10) de méthanation selon l'une des revendications 10 ou 11, dans lequel chaque composé à base de carbone est un gaz, le réacteur comportant au moins une buse d'injection d'eau (120) et au moins une buse d'injection d'un gaz comportant le gaz à base de carbone et du dihydrogène (110), au moins une buse d'injection d'eau étant positionnée en dessous d'au moins une buse d'injection du gaz.

14. Réacteur (10) de méthanation selon l'une des revendications 10 à 13, qui comporte, en aval de la sortie de méthane et d'eau (115), un moyen de séparation gaz - solide (135).

15. Réacteur (10) de méthanation selon l'une des revendications 10 à 14, qui comporte en aval de la sortie de méthane et d'eau (115) un échangeur (145) de chaleur configuré pour refroidir le méthane et l'eau et pour co-générer de l'énergie thermique au cours de l'échange de chaleur réalisé.

## Patentansprüche

1. Methanisierungsverfahren (20) zur Reaktion von molekularem Wasserstoff in gasförmiger Form mit mindestens einer Verbindung auf Kohlenstoffbasis in gasförmiger Form und Produktion des Methans, das aufweist:
- einen Schritt (210) des Eintretens jeder Verbindung auf Kohlenstoffbasis und des molekularen Wasserstoffs in einen Hohlkörper (105), der für den Empfang eines Fließbetts katalytischer Partikel (106) ausgelegt ist,
- einen Methanisierungsreaktionsschritt (215) zwischen dem Wasserstoff und jeder Verbindung auf Kohlenstoffbasis und
- einen Schritt (220) des Austretens von Methan und von Wasser;
**dadurch gekennzeichnet, dass** es weiterhin aufweist:
- einen Schritt (205) des Einleitens von Kühlwasser in flüssiger Phase in das Fließbett beim Methanisierungsreaktionsschritt,
- einen Schritt des Erfassens der Temperatur in dem Reaktor und
- einen Schritt der Regulierung der in den Hohlkörper eingeleiteten Wassermenge in Abhängigkeit von der beim Temperaturerfassungsschritt gemessenen Temperatur.

2. Methanisierungsverfahren (20) nach Anspruch 1, wobei beim Schritt (210) des Eintretens jeder Verbindung auf Kohlenstoffbasis und des molekularen Wasserstoffs dieses Eintreten in das Fließbett katalytischer Partikel (106) durchgeführt wird.

3. Methanisierungsverfahren (20) nach Anspruch 2, wobei beim Schritt (205) des Einleitens von Wasser das Wasser näher zur Basis des Hohlkörpers (105) als zum Eintritt jeder Verbindung auf Kohlenstoffbasis und des molekularen Wasserstoffs (110) eingeleitet wird.

4. Methanisierungsverfahren (20) nach einem der Ansprüche 2 oder 3, wobei jede Verbindung auf Kohlenstoffbasis ein Gas ist, der Schritt (210) des Eintretens jeder Verbindung auf Kohlenstoffbasis und des molekularen Wasserstoffs in einen Hohlkörper (105) über mindestens eine Gaseinleitungsdüse durchgeführt wird und der Schritt (205) des Einleitens von Wasser über eine Wassereinleitungsdüse durchgeführt wird, die unterhalb mindestens einer Gaseinleitungsdüse positioniert ist.

5. Methanisierungsverfahren (20) nach einem der Ansprüche 1 bis 4, das beim Schritt (220) des Austretens von Methan und von Wasser einen Kondensationsschritt von aus dem Hohlkörper (105) austretendem Wasserdampf aufweist.

6. Methanisierungsverfahren (20) nach Anspruch 5, das beim Schritt (205) des Einleitens von kondensiertem Wasser einen Einleitungsschritt des kondensierten Wassers in das Fließbett aufweist.

7. Methanisierungsverfahren (20) nach einem der Ansprüche 1 bis 6, das nach dem Schritt (220) des Austretens von Methan und von Wasser einen Gas-Festkörper-Trennschritt aufweist.

8. Methanisierungsverfahren (20) nach einem der Ansprüche 1 bis 7, das nach dem Schritt (220) des Austretens von Methan und von Wasser einen Schritt des Austauschs von Wärme aufweist, bei dem das Methan und das Wasser gekühlt werden und gleichzeitig thermische Energie erzeugt wird.

9. Methanisierungsverfahren (20) nach einem der Ansprüche 1 bis 8, wobei die Menge des beim Schritt (205) des Einleitens von Kühlwasser in flüssiger Phase in den Hohlkörper (105) eingeleiteten Wassers über 75 % der Wassermenge beträgt, die beim Schritt (220) des Austretens von Methan und von Wasser aus dem Hohlkörper austritt.

10. Methanisierungsreaktor (10), der das Methanisierungsverfahren (20) nach Anspruch 6 durchführt, indem er molekularen Wasserstoff in gasförmiger Form mit mindestens einer Verbindung auf Kohlenstoffbasis in gasförmiger Form in Reaktion versetzt und Methan produziert, der aufweist:
- einen Hohlkörper (105), der für den Empfang eines Fließbetts katalytischer Partikel (106) ausgelegt ist und einen Eintritt (110) jeder Verbindung auf Kohlenstoffbasis und des molekularen Wasserstoffs aufweist und
- einen Methan- und Wasseraustritt (115),
**dadurch gekennzeichnet, dass** er weiterhin aufweist:
- einen Eintritt (120) für das Einleiten von Kühlwasser in flüssiger Phase in das Fließbett,
- einen Temperatursensor (107) im Reaktor,
- ein Mittel (140) für die Regulierung der in den Hohlkörper in Abhängigkeit von der vom Temperatursensor gemessenen Temperatur eingeleiteten Wassermenge,
- ein dem Methan- und Wasseraustritt (115) nachgelagertes Wasserdampfkondensationsmittel (125) und
- einen Kreis (130) für den Transport von kondensiertem Wasser bis zum Eintritt (120) für das Einleiten von Kühlwasser.

11. Methanisierungsreaktor (10) nach Anspruch 10, wobei der Eintritt (110) jeder Verbindung auf Kohlenstoffbasis und des molekularen Wasserstoffs im Bett (106) durchgeführt wird.

12. Methanisierungsreaktor (10) nach Anspruch 11, wobei der Wassereintritt (120) näher an der Basis des Hohlkörpers (105) ist als der Eintritt jeder Verbindung auf Kohlenstoffbasis und des molekularen Wasserstoffs (110).

13. Methanisierungsreaktor (10) nach einem der Ansprüche 10 oder 11, wobei jede Verbindung auf Kohlenstoffbasis ein Gas ist, wobei der Reaktor mindestens eine Wassereinleitungsdüse (120) und mindestens eine Düse zum Einleiten eines Gases aufweist, das das Gas auf der Basis von Kohlenstoff und des molekularen Wasserstoffs (110) aufweist, wobei mindestens eine Wassereinleitungsdüse unterhalb von mindestens einer Gaseinleitungsdüse positioniert ist.

14. Methanisierungsreaktor (10) nach einem der Ansprüche 10 bis 13, der dem Methan- und Wasseraustritt (115) nachgelagert ein Gas-Feststoff-Trennmittel aufweist.

15. Methanisierungsreaktor (10) nach einem der Ansprüche 10 bis 14, der dem Methan- und Wasseraustritt (115) nachgelagert einen Wärmetauscher (145) aufweist, der ausgelegt ist, um das Methan und das Wasser zu kühlen und um gleichzeitig beim durchgeführten Wärmetausch thermische Energie zu erzeugen.

## Claims

1. Methanation method (20) for reacting hydrogen in gaseous form with at least one carbon-based compound in gaseous form and producing methane, which comprises:
- a step (210) of inputting each carbon-based compound and hydrogen into a hollow body (105) configured to receive a fluidized bed of catalytic particles (106);
- a step (215) of methanation reaction between the hydrogen and each carbon-based compound; and
- a step (220) of methane and water exiting;
**characterized in that** it also comprises:
- a step (205) of injecting liquid-phase cooling water into the fluidized bed during the methanation reaction step;
- a step of capturing the temperature in the reactor; and
- a step of regulating the flow rate of the water introduced into the hollow body as a function of the temperature measured during the temperature capture step.

2. Methanation method (20) according to claim 1 wherein, during the step (210) of inputting each carbon-based compound and hydrogen, this input is performed in the fluidized bed of catalytic particles (106).

3. Methanation method (20) according to claim 2 wherein, during the water injection step (205), the water is injected closer to the base of the hollow body (105) than the inlets of each carbon-based compound and of the hydrogen (110).

4. Methanation method (20) according to one of claims 2 or 3, wherein each carbon-based compound is a gas, the step (210) of inputting each carbon-based compound and hydrogen into a hollow body (105) is performed by means of at least one gas injection nozzle, and the water injection step (205) is performed by means of a water-injection nozzle positioned below at least one gas-injection nozzle.

5. Methanation method (20) according to one of claims 1 to 4, which comprises a step of condensing the water vapor output from the hollow body (105) during the step (220) of outputting methane and water.

6. Methanation method (20) according to claim 5, which comprises a step of injecting condensed water into the fluidized bed during the condensed water injection step (205).

7. Methanation method (20) according to one of claims 1 to 6, which comprises a gas-solid separation step after the step (220) of outputting methane and water.

8. Methanation method (20) according to one of claims 1 to 7, which comprises a heat exchange step of cooling the methane and the water and co-generating thermal energy after the step (220) of outputting methane and water.

9. Methanation method (20) according to one of claims 1 to 8, wherein the amount of water introduced into the hollow body (105) during the step (205) of injecting liquid-phase cooling water is greater than 75% of the amount of water output from the hollow body during the step (220) of outputting methane and water.

10. Methanation reactor (10) utilizing the methanation method (20) according to claim 6 by reacting hydrogen in gaseous form with at least one carbon-based compound in gaseous form and producing methane, which comprises:
- a hollow body (105) configured to receive a fluidized bed of catalytic particles (106) and comprising an inlet (110) for each carbon-based compound and for hydrogen; and
- an outlet (115) for methane and water;
**characterized in that** it also comprises:
- an inlet (120) for the injection of liquid-phase cooling water into the fluidized bed;
- a temperature sensor (107) in the reactor;
- a means (140) for regulating the flow rate of the water introduced into the hollow body as a function of the temperature measured by the temperature sensor;
- a means (125) for condensing water vapor present downstream of the outlet (115) for methane and water; and
- a circuit (130) for transporting condensed water to the inlet (120) for injecting cooling water.

11. Methanation reactor (10) according to claim 10, wherein the input (110) of each carbon-based compound and the hydrogen is realized in the bed (106).

12. Methanation reactor (10) according to claim 11, wherein the water inlet (120) is closer to the base of the hollow body (105) than the inlets of each carbon-based compound and of the hydrogen (110).

13. Methanation reactor (10) according to one of claims 10 or 11, wherein each carbon-based compound is a gas, the reactor comprising at least one water-injection nozzle (120) and at least one injection nozzle for a gas comprising the carbon-based gas and hydrogen (110), at least one water-injection nozzle being positioned below at least one gas-injection nozzle.

14. Methanation reactor (10) according to one of claims 10 to 13, which comprises, downstream of the outlet (115) for methane and water, a gas-solid separation means (135).

15. Methanation reactor (10) according to one of claims 10 to 14, which comprises, downstream of the outlet (115) for methane and water, a heat exchanger (145) configured to cool the methane and water and co-generate thermal energy during the heat exchange realized.
